# EUROPÄISCHE PATENTANMELDUNG

(11) **EP 4 257 175 A1**
(43) Veröffentlichungstag der Anmeldung: **11.10.2023**
(21) Anmeldenummer: 22020150.3
(22) Anmeldetag: 04.04.2022
(51) Int. Cl.: A61N 1/05, A61N 1/36

(54) **ELEKTRODENARRAY UND ELEKTRODE FÜR DIE PERIPHERE NERVENSTIMULATION**

(71) Anmelder: Aurimod GmbH, 1030 Wien (AT)
(72) Erfinder: Kampusch, Stefan, 1180 Wien (AT)
(74) Vertreter: Keschmann, Marc

(57) **Zusammenfassung**

Ein Elektrodenarray (5) für die periphere Nervenstimulation, insbesondere für die aurikuläre Punktualstimulation des Vagusnerv, umfasst einen plattenförmigen Träger (14) und eine Vielzahl von vom Träger (14) vorstehenden nadelförmigen Elektroden (15) und wenigstens einen mit den Elektroden (15) oder einer Untergruppe von Elektroden (15) elektrisch leitend verbundenen elektrischen Anschluss für die elektrische Verbindung mit einer Stimulationsvorrichtung, wobei die Elektroden (15) eine maximale Länge von 0,6mm, bevorzugt 0,4mm aufweisen.

## Beschreibung

Die Erfindung betrifft ein Elektrodenarray für die periphere Nervenstimulation, insbesondere für die aurikuläre Punktualstimulation z.B. des Vagusnerv.

Die Erfindung betrifft weiters eine Elektrode für die periphere Nervenstimulation, insbesondere für die aurikuläre Punktualstimulation z.B. des Vagusnerv.

Die Erfindung betrifft weiters eine Vorrichtung zur peripheren Nervenstimulation, insbesondere zur aurikulären Punktualstimulation z.B. des Vagusnerv eines Patienten, umfassend einen Stromgenerator zur Erzeugung von Stimulationsstromimpulsen und wenigstens ein Elektrodenarray oder wenigstens eine Elektrode, das bzw. die mittels einer elektrischen Leitung mit dem Stromgenerator verbunden ist.

Verfügbare Stimulationsgeräte für die elektrische Stimulation des aurikulären Vagusnervs werden in der Therapie verschiedenster Erkrankungen, wie z.B. chronischer und akuter Schmerzen, Epilepsie und Depression, eingesetzt. Bei aurikulären Punktualstimulationsgeräten, wie sie z.B. aus der WO 2011/030210 A1 bekannt geworden sind, werden elektrische Stimulationspulse über Nadelelektroden eingeleitet, die an vorgegebenen Stellen der Ohrmuschel in die Haut eingestochen werden und dort für den Behandlungszeitraum von z.B. mehreren Tagen verbleiben.

Die Stimulation ermöglicht zum Beispiel die vorteilhafte Beeinflussung von Schmerzverarbeitung und Schmerzwahrnehmung. Auch wird durch die Stimulation die sympathovagale Balance im autonomen Nervensystem positiv beeinflusst. In der Folge verändert sich der aktuelle physiologische Zustand des Patienten dynamisch, gemessen als Veränderung der Herzrate, der Atemrate, des Blutdrucks, der lokalen Durchblutung und anderer Parameter.

Stimulationsgeräte für periphere Nervenstimulation verwenden zumeist Oberflächenelektroden. Dabei handelt es sich z.B. um Ballelektroden, die auf der Haut anliegen oder durch mechanische Passung, z.B. mit der Form der Ohrmuschel, an die Haut angepresst werden. Im Anwendungsbereich der transkutanen Nervenstimulation gibt es auch Klebeelektroden, die meist großflächig auf den zu stimulierenden Hautbereich aufgeklebt werden. Die Stimulation erfolgt so über einen größeren Hautbereich, da die Elektroden eine gewisse Größe aufweisen müssen, um keine Verbrennungen zu verursachen, da hohe Stimulationsenergien verwendet werden müssen, um den Hautwiderstand zu überwinden. Eine Fixierung der Elektroden, abgesehen von Klebeelektroden, ist hier oft schwierig und beschränkt die Anwendung meist auf eine intermittierende, nicht chronische Anwendung. Ein weiterer Nachteil großflächiger Oberflächenelektroden liegt in der örtlich unspezifischen Stimulation.

Andererseits gibt es Anwendungen der perkutanen Stimulation, wo mittels kleiner Nadelelektroden bis zu 2 mm tief in die Haut eingestochen wird, um dort Nervenfasern zu reizen. Mit solchen Nadelelektroden kann man sehr spezifisch stimulieren und überwindet den Hautwiderstand mit resultierender niedrigerer Stimulationsenergie im Vergleich zu Oberflächenelektroden, da der Hautwiderstand nicht mehr relevant ist. Auch hier ist die Fixierung nicht einfach, aber leichter möglich als bei Oberflächenelektroden, was eine chronische Anwendung über mehrere Tage prinzipiell erlaubt. Allerdings schmerzen längere Nadelelektroden beim Einstechen und können teilweise nur schwer für längere Dauer fixiert werden. Das meist elastische Gewebe drückt die Nadelelektroden mit der Zeit wieder aus dem Gewebe heraus.

Grundsätzlich ist man bestrebt, die Stimulationsenergie möglichst zu reduzieren, um eine miniaturisierte Bauweise und eine hohe Energieeffizienz von eingesetzten Stimulationsgeräten zu erzielen.

Die vorliegende Erfindung zielt daher darauf ab, die obigen Nachteile zu überwinden und eine Elektrodenanordnung für die periphere Nervenstimulation zu schaffen, die eine örtlich spezifische Stimulation erlaubt, eine langfristige Behandlung ermöglicht, den Tagekomfort erhöht und die erforderliche Stimulationsenergie minimiert. Weiters zielt die Erfindung darauf ab, ein Elektrodenarray oder eine einzelne Elektrode dahingehend zu verbessern, dass sein bzw. ihr Halt im Gewebe verbessert und sein bzw. ihr Widerstand gegen ein Herausziehen oder Herausfallen erhöht wird.

Zur Lösung dieser Aufgabe besteht die Erfindung gemäß einem ersten Aspekt in der Breitstellung eines Elektrodenarrays für die periphere Nervenstimulation, insbesondere für die aurikuläre Punktualstimulation z.B. des Vagusnerv, umfassend einen plattenförmigen Träger und eine Vielzahl von vom Träger vorstehenden nadelförmigen Elektroden und wenigstens einen mit den Elektroden oder einer Untergruppe von Elektroden elektrisch leitend verbundenen elektrischen Anschluss für die elektrische Verbindung mit einer Stimulationsvorrichtung, wobei die Elektroden eine maximale Länge von 0,6mm, bevorzugt 0,4-0,6mm aufweisen.

Die Erfindung beruht somit auf dem Ansatz, anstelle einer einzelnen langen Nadel oder einer Oberflächenelektrode ein Elektrodenarray zu verwenden, das eine Mehrzahl von kurzen Elektroden aufweist, die zwar in das Gewebe eindringen, dies jedoch in einem begrenzten Ausmaß. Die Elektroden weisen hierbei eine maximale Länge von 0,6mm, bevorzugt 0,4-0,6mm, auf, wobei unter der Länge der Elektroden der Abstand zwischen der Nadelspitze und der die Hautauflagefläche definierenden Oberfläche des plattenförmigen Trägers zu verstehen ist. Die Elektrodenlänge kann daher mit der maximalen Eindringtiefe der Elektroden in die Haut eines Patienten gleichgesetzt werden.

So können die Stimulationsimpulse einerseits ortsspezifisch eingebracht werden und es kann andererseits die Stimulationsenergie niedrig gehalten werden. Weiters wird die Invasivität im Vergleich zu langen Nadelelektroden reduziert. Die Aufbauhöhe kann dadurch stark reduziert und damit die mechanische Stabilität erhöht werden. Des Weiteren führen mehrere kleine Nadelelektroden zu einem besseren Sitz im Gewebe. Schmerzen beim Einstechen werden nahezu vollständig verhindert. Der Tragekomfort wird wesentlich erhöht. Für die chronische Anwendung über mehrere Tage ist die Fixierung der Elektroden wesentlich einfacher möglich.

Auf Grund der niedrigeren Stimulationsenergie und der erhöhten Oberfläche durch multiple Nadeln ist weiters die Gefahr reduziert, im Betrieb diejenige Stromdichte ("Charge Injection Limit") an der Elektrode zu überschreiten, unterhalb der die Stimulationspulse keine Reaktionsprodukte erzeugen und sohin keine Korrosionsprozesse an der Elektrode stattfinden lassen.

Hinsichtlich der Anordnung der Elektroden auf dem plattenförmigen Träger sind grundsätzliche keine Einschränkungen vorgesehen. Eine möglichst gleichmäßige Verteilung der Stimulationsenergie über den vom Elektrodenarray abgedeckten Bereich des Gewebes gelingt gemäß einer bevorzugten Ausbildung der Erfindung jedoch dadurch, dass die Elektroden in einem regelmäßigen Raster auf dem Träger angeordnet sind.

Bevorzugt sind die Elektroden in einem Abstand von 0,5-1mm voneinander angeordnet, wobei hier jeweils der kürzeste Abstand zwischen zwei benachbarten Elektroden herangezogen wird.

Das Elektrodenarray kann mindestens drei Elektroden umfassen. Bevorzugt umfasst das Elektrodenarray mindestens 9, bevorzugt mindestens 16, bevorzugt mindestens 25 Elektroden.

Hinsichtlich der Formgebung der Elektroden ist bevorzugt vorgesehen, dass es sich um spitz zulaufende Nadelelektroden handelt, wodurch eine einfache und möglichst schmerzfreie Penetration der Hautoberfläche gewährleistet ist. Insbesondere können die Elektroden kegelförmig ausgebildet sein, wobei die Kegelform lediglich an der Elektrodenspitze vorgesehen sein kann oder die Elektrode über ihre gesamte Länge der Kegelform entspricht.

Eine optimierte Elektrodenform ergibt sich gemäß einer bevorzugten Weiterbildung dadurch, dass die Elektroden in einem mittleren Bereich eine Verdickung aufweisen, an welche sich eine kegelförmige Spitze anschließt. Die im mittleren Bereich vorgesehene Verdickung erhöht die Ausziehkraft, sodass das Elektrodenarray insgesamt fester und stabiler im Gewebe festhält.

Die genannte optimierte Elektrodenform bildet einen unabhängigen, zweiten Aspekt der Erfindung, wonach eine Elektrode für die periphere Nervenstimulation, insbesondere für die aurikuläre Punktualstimulation z.B. des Vagusnerv, in einem mittleren Bereich eine Verdickung aufweist, an welche sich eine kegelförmige Spitze anschließt. Bevorzugt weist die Elektrode hierbei einen insbesondere zylindrischen Basisabschnitt auf, dessen Durchmesser geringer ist als der des verdickten mittleren Bereichs. Dieser zweite Aspekt der Erfindung ist nicht auf ein Elektrodenarray beschränkt, sondern betrifft insbesondere eine einzelne Elektrode.

Weiters kann bevorzugt vorgesehen sein, dass die Nadelspitze der Elektroden einen Durchmesser von 0,02-0,04mm aufweist. Dies führt zu entsprechend scharfen Spitzen, die ohne Mühe in die Haut eindringen können.

Das erfindungsgemäße Elektrodenarray kann aus verschiedensten Materialien hergestellt sein. Gemäß einer ersten Alternative besteht das Elektrodenarray im Wesentlichen vollständig aus einem elektrisch leitenden Material, insbesondere aus einem Metall, wie z.B. Titan oder Platin-Iridium oder aus einem leitfähigen Kunststoff, wie z.B. Poly-3,4-ethylendioxythiophen.

Gemäß einer zweiten Alternative bestehen zumindest die Elektroden aus einem elektrisch leitenden Material, wie z.B. Titan oder Platin-Iridium oder aus einem leitfähigen Kunststoff, wie z.B. Poly-3,4-ethylendioxythiophen, und der Träger besteht aus einem elektrisch nicht-leitenden Material, insbesondere einem Polymer.

Gemäß einer weitere Alternative kann das Elektrodenarray aus einem elektrisch nicht-leitenden Material bestehen und die Elektroden sind mit einer elektrisch leitenden Beschichtung z.B. aus Gold oder Titan versehen. Die Beschichtung kann hierbei durch beliebige Beschichtungsverfahren aufgebracht werden, wie z.B. durch Sputtern oder galvanische Beschichtung.

Die Herstellung des Elektrodenarrays kann durch Fräsen aus einem Vollkörper, durch ein Gussverfahren, wie z.B. Spritzguss, durch Stanzen aus einer Platte oder durch ein additives Fertigungsverfahren (3D-Druck) erfolgen. Die additive Fertigung kann beispielswiese mittels eines stereolithografischen Druckverfahrens vorgenommen werden, wodurch eine hohe räumliche Auflösung erzielt werden kann und die Elektroden entsprechend präzise, formgenau und mit glatter Oberfläche hergestellt werden können. Das Elektrodenarray kann beispielsweise aus einem leitfähigen Kunststoff oder aus einem metallischen und daher elektrisch leitfähigen Material gedruckt werden. Alternativ kann das Elektrodenarray aus einem nicht leitfähigen Material gedruckt und dann mit einen leitfähigen Material beschichtet werden.

Der plattenartige Träger des erfindungsgemäßen Elektrodenarrays kann als starrer Träger oder als flexible Struktur, wie z.B. als Folie oder als Blech ausgebildet sein. Alternativ kann der Träger als leitfähiges Gelpad (z.B. leitfähiges Hydrogel) ausgebildet sein, das mit den Elektrodennadeln durchstochen wird und so eine flexible Elektrode bildet. Die Ausbildung als flexible Struktur erlaubt eine einfache Anpassung des Trägers an die jeweilige Oberflächenkontur derjenigen Körperstelle, auf welche das Elektrodenarray aufgebracht werden soll. Im Falle einer Ausbildung als starrer Träger, kann der Träger eine Hautkontaktoberfläche bereitstellen, von welcher die Elektroden vorragen, die eben ausgebildet ist. Alternativ kann die Hautkontaktoberfläche des Trägers an die gewünschte Körperstelle formangepasst sein.

Zur Verbesserung der Befestigung des Elektrodenarrays an der Haut eines Patienten kann die Hautkontaktoberfläche des Trägers mit einer Klebebeschichtung versehen sein.

Zur Stimulation der peripheren Nervenfasern an einer bestimmten Körperstelle eines Patienten können mehrere der erfindungsgemäßen Elektrodenarrays zum Einsatz gelangen, von denen jedes an einen eigenen Stimulationskanal einer zugeordneten Stimulationsvorrichtung angeschlossen ist. Alternativ kann das erfindungsgemäße Elektrodenarray auch als großflächigere Matrize ausgeformt und verwendet werden, die mehrere Stimulationskanäle unterstützt. Auf diese Weise kann bipolar und mono-, bi- oder multi-phasisch stimuliert werden.

Im Falle eines großflächigeren Elektrodenarrays, das zwei oder mehrere Stimulationskanäle unterstützt, sieht eine bevorzugte Weiterbildung der Erfindung vor, dass die Elektroden wenigstens eine erste und eine zweite Gruppe von Elektroden aufweisen, wobei jede Gruppe von Elektroden mit einem eigenen elektrischen Anschluss elektrisch leitend verbunden ist.

Gemäß einem weiteren Aspekt der Erfindung wird eine Vorrichtung zur peripheren Nervenstimulation, insbesondere zur aurikulären Punktualstimulation z.B. des Vagusnerv eines Patienten bereitgestellt, umfassend einen Stromgenerator zur Erzeugung von Stimulationsstromimpulsen und wenigstens ein Elektrodenarray gemäß dem ersten Aspekt der Erfindung oder wenigstens zwei Elektroden nach dem zweiten Aspekt der Erfindung, das bzw. die mit jeweils wenigstens einer elektrischen Leitung mit dem Stromgenerator verbunden ist bzw. sind.

Für die periphere Nervenstimulation ist es besonders vorteilhaft, wenn der Stromgenerator zur Erzeugung der Stimulationsstromimpulse mit einer Pulsfrequenz von < 1kHz ausgebildet ist.

Hinsichtlich der Stromstärke sieht eine bevorzugte Weiterbildung vor, dass der Stromgenerator zur Erzeugung der Stimulationsstromimpulse mit einer Stromamplitude von > 5mA ausgebildet ist. Die Stimulationsstromimpulse können hierbei bevorzugt eine alterierende Polarität aufweisen.

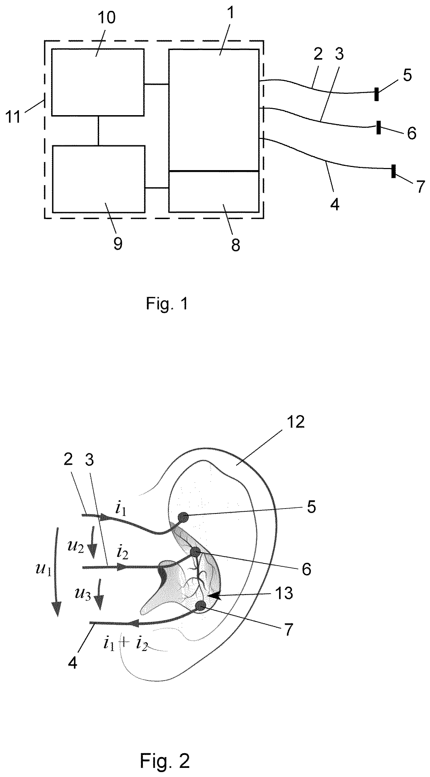

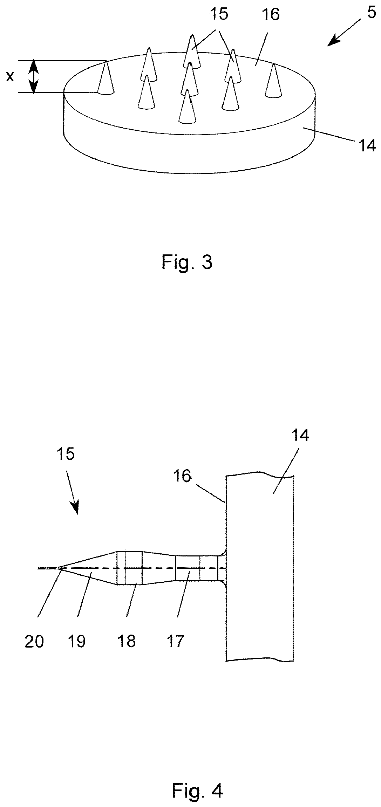

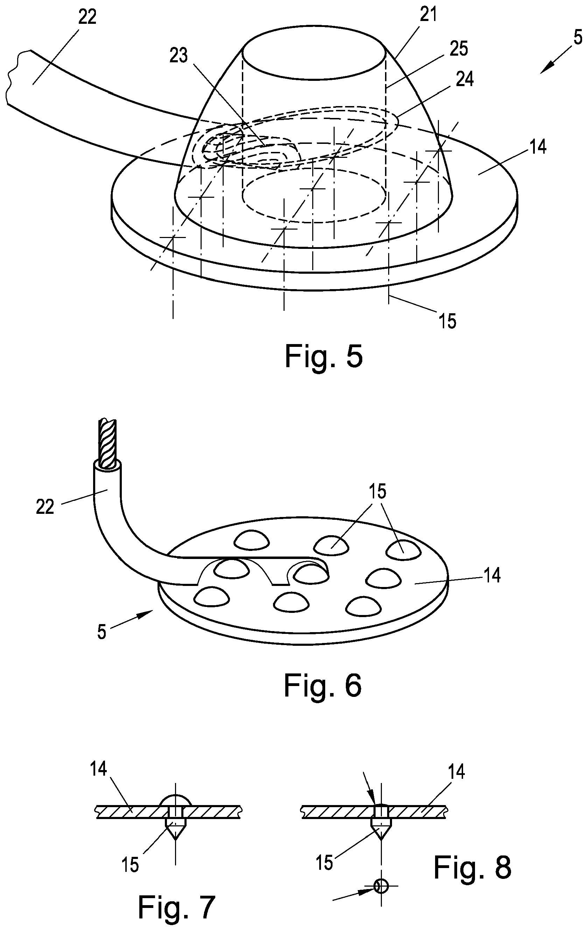

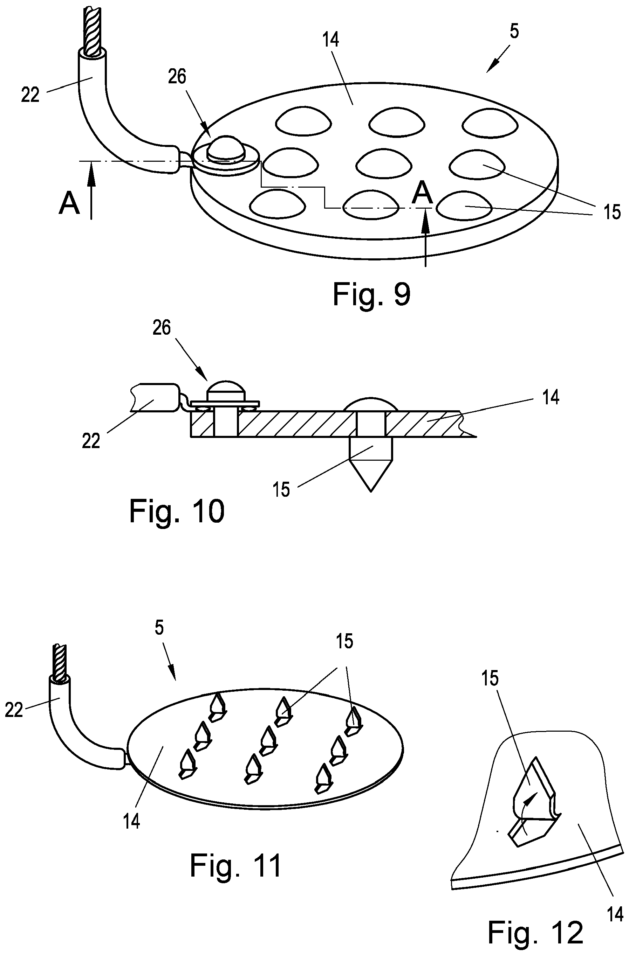

Die Erfindung wird nachfolgend anhand von in der Zeichnung schematisch dargestellten Ausführungsbeispielen näher erläutert. In dieser zeigen Fig. 1 eine schematische Darstellung einer erfindungsgemäßen Vorrichtung, Fig. 2 die Anordnung von Nadelelektroden an einem menschlichen Ohr mit einer Darstellung von Spannung und Strom für die Vagusnervstimulation, Fig. 3 eine schematische Darstellung eines erfindungsgemäßen Elektrodenarrays, Fig. 4 eine Ansicht einer beispielhaften Form einer Elektrode, Fig. 5 eine schematische Darstellung eines Elektrodenarrays mit einem elektrischen Anschluss für eine Stimulationsstromleitung, Fig. 6 eine alternative Ausbildung eines Elektrodenarrays, Fig. 7 eine Detaildarstellung einer Elektrodenbefestigung, Fig. 8 eine alternative Ausführung der Elektrodenbefestigung, Fig. 9 eine weitere alternative Ausbildung eines Elektrodenarrays, Fig. 10 einen Schnitt entlang der Linie A-A der Fig. 9, Fig. 11 eine weitere alternative Ausbildung eines Elektrodenarrays und Fig. 12 eine Detaildarstellung einer Elektrode des Elektrodenarrays von Fig. 11.

Fig. 1 zeigt eine beispielhafte Ausführung der erfindungsgemäßen Vorrichtung umfassend einen von einer nicht dargestellten Batterie gespeisten Strom- oder Spannungsgenerator 1, an den elektrische Leitungen 2, 3 und 4 angeschlossen sind, an deren Enden je ein am Ohr zu positionierendes Elektrodenarray 5, 6 bzw. 7 angeordnet ist. Der Generator 1 ist zur Erzeugung von Stimulationsstromimpulsen ausgebildet, die über die Leitungen 2, 3, 4 und die zugehörigen Elektrodenarrays 5, 6, 7 in z.B. das Ohr eingebracht werden. Zur Messung der z.B. über die Elektrodenarrays 6 und 7 abgegriffenen Gewebeimpedanz ist eine Messschaltung 8 vorgesehen.

Mit der Messschaltung 8 ist eine Signalverarbeitungsschaltung 9 verbunden, der die Messwerte der Messschaltung 8 zugeführt sind und die ausgebildet ist, um aus dem zeitlichen Verlauf der Gewebeimpedanz wenigstens einen physiologischen Messwert, wie z.B. die Herzrate, die Herzratenvariabilität, die Durchblutung, die Gefäßsteifigkeit und/oder die Atemfrequenz, zu ermitteln. Weiters ist eine Steuerschaltung 10 vorgesehen, die mit dem Stromgenerator 1 zur Änderung wenigstens eines Stimulationsstromparameters, wie z.B. der Pulsfrequenz und/oder der Stromamplitude der Stimulationsstromimpulse, zusammenwirkt. Die Veränderung des wenigstens einen Stimulationsstromparameters kann hierbei in Abhängigkeit des von der Signalverarbeitungsschaltung 9 ermittelten physiologischen Messwerts bzw. von dessen zeitlichen Verlauf erfolgen, zu welchem Zweck der Messwert der Steuerschaltung 10 von der Signalverarbeitungsschaltung 9 zugeführt wird.

Der Stromgenerator 1, die Messschaltung 8, die Signalverarbeitungsschaltung 9 und die Steuerschaltung 10 sind in einem Gehäuse 11 angeordnet, das in der Nähe des Ohrs, z.B. hinter dem Ohr, angebracht werden kann. Der Stromgenerator 1, die Messschaltung 8, die Signalverarbeitungsschaltung 9 und die Steuerschaltung 10 können als gesonderte Baueinheiten ausgebildet oder in einer gemeinsamen elektronischen Schaltung implementiert sein.

Fig. 2 zeigt das menschliche Ohr 12 mit Blutgefäßen und den afferenten Vagusnerv-Ästen 13. Im Bereich der Vagusnerv-Äste sind die Elektrodenarrays 5, 6 und 7 in das Gewebe eingestochen, wobei über die Leitung 2 eine Folge von Stimulationsimpulsen mit dem Strom *i₁* und über die Leitung 3 eine Folge von Stimulationsimpulsen mit dem Strom *i₂* eingebracht werden und der Stromrückfluss *i₁+i₂* über die Leitung 4 erfolgt. Es ergibt sich zwischen den Leitungen 2 und 4 eine Spannung *u₁*, zwischen den Leitungen 2 und 3 eine Spannung *u₂* und zwischen den Leitungen 3 und 4 eine Spannung *u₃*. Alternativ kann auch eine konstante Spannung eingeprägt und der Strom gemessen werden.

Fig. 3 zeigt ein Elektrodenarray 5, wobei die Elektrodenarrays 6 und 7 ähnlich aufgebaut sein können. Das Elektrodenarray 5 umfasst einen plattenförmigen Träger 14, an dem eine Mehrzahl von Nadelelektroden 15 rasterartig angeordnet ist. Die Nadelelektroden 15 weisen eine von der hautzugewandten Oberfläche 16 des Trägers 14 gemessene Länge x von z.B. 0,2-0,6mm auf und durchringen die Hautoberfläche eines Patienten. Bei der in Fig. 3 dargestellten Ausführungsform sind die Nadelelektroden 15 kegelförmig ausgebildet.

Fig. 4 zeigt eine alternative Form einer Nadelelektrode 15, welche einen unabhängigen Gegenstand der vorliegenden Erfindung bildet. Die Nadelelektrode kann entweder als Teil eines Elektrodenarrays 5 oder als Einzelnadel verwendet werden. Die Nadelelektrode 15 umfasst einen im Wesentlichen zylindrischen Basisabschnitt 17 eine verdickten mittleren Abschnitt 18 und eine konisch zulaufenden Nadelabschnitt 19 mit einer Nadelspitze 20.

Fig. 5 zeigt ein Elektrodenarray 5 der in Fig. 3 gezeigten Art mit einem Träger 14 und nur schematisch angedeuteten Nadelelektroden 15. An der den Nadelelektroden 15 abgewandten Seite des Trägers 14 ist ein Leitungsanschluss vorgesehen, welcher der elektrisch leitenden Verbindung eines abisolierten Endes 23 eines Stromleiters 22 mit dem Elektrodenarray 5 dient. Der Leitungsanschluss weist zu diesem Zweck ein im Wesentlichen zylindrisches Anschlussteil 25 auf, das am Träger 14 angeordnet ist. Zum elektrischen Verbinden des Endes 23 mit dem Anschlussteil 25 wird an das Ende 23 ein ringförmiges Klemmteil 24 angebracht, z.B. durch Löten oder durch Anspritzen des aus leitfähigem Kunststoff bestehenden Klemmteils. Das Klemmteil wird über das zylindrische Anschlussteil 25 geschoben und z.B. durch eine Rast- oder Klemmverbindung befestigt, wodurch eine elektrisch leitende Verbindung zwischen dem Ende 23 bzw. dem Klemmteil 24 und dem Anschlussteil 25 hergestellt wird. Anschließend kann der Leitungsanschluss mit einer Abdeckung, Hülle oder Verkapselung 21 versehen werden, welche z.B. durch Umspritzen aus einem Kunststoff hergestellt werden kann.

Bei der alternativen Ausbildung gemäß Fig. 6 erfolgt der Anschluss des Stromleiters 22 an das Elektrodenarray 5 durch Befestigung an eine der Nadelelektroden 15. Die Befestigung kann beispielsweise durch Klemmen, Nieten oder Schweißen erfolgen, je nachdem wie die einzelnen Nadelelektroden 15 am Träger 14 befestigt sind. Bei der Ausführung gemäß Fig. 7 sind die Nadelelektroden 15 mit dem Träger 14 vernietet. Hierbei kann der Stromleiter 22 zwischen dem verdickten Kopf der Nadelelektrode 15 und dem Träger 14 geklemmt werden. Bei der Ausführung gemäß Fig. 8 sind die Nadelelektroden 15 mit dem Träger 14 verschweißt. Hierbei kann der Stromleiter 22 mittels eines Schweißpunkts mit der Nadelelektrode 15 oder dem Träger 14 verbunden werden.

Bei der in Fig. 9 und 10 dargestellten alternativen Ausführungsform ist der Stromleiter 22 an einem eigenen Befestigungspunkt 26 mit dem Träger 14 elektrisch leitend verbunden. Die Befestigung kann hierbei z.B. mittels eines Klemmstifts erfolgen.

Fig. 11 und 12 zeigen eine alternative Ausgestaltung des Elektrodenarrays 5, bei dem die Nadelelektroden 15 einstückig mit dem Träger 14 ausgebildet sind und durch Stanzen aus dem plattenförmigen Träger 14 herausgebogen wurden. Hierbei kann der Stromleiter 22 beim Stanzvorgang direkt mit einprägt werden.

## Patentansprüche

1. Elektrodenarray für die periphere Nervenstimulation, insbesondere für die aurikuläre Punktualstimulation z.B. des Vagusnerv, umfassend einen plattenförmigen Träger (14) und eine Vielzahl von vom Träger (14) vorstehenden nadelförmigen Elektroden (15) und wenigstens einen mit den Elektroden (15) oder einer Untergruppe von Elektroden (15) elektrisch leitend verbundenen elektrischen Anschluss für die elektrische Verbindung mit einer Stimulationsvorrichtung, wobei die Elektroden (15) eine maximale Länge von 0,6mm, bevorzugt 0,4-0,6mm aufweisen.

2. Elektrodenarray nach Anspruch 1, **dadurch gekennzeichnet, dass** die Elektroden (15) in einem regelmäßigen Raster auf dem Träger (14) angeordnet sind.

3. Elektrodenarray nach Anspruch 1 oder 2, **dadurch gekennzeichnet, dass** die Elektroden (15) in einem Abstand von 0,5-1mm voneinander angeordnet sind.

4. Elektrodenarray nach Anspruch 1, 2 oder 3, **dadurch gekennzeichnet, dass** die Elektroden (15) kegelförmig ausgebildet sind.

5. Elektrodenarray nach einem der Ansprüche 1 bis 4, **dadurch gekennzeichnet, dass** die Elektroden (15) in einem mittleren Bereich eine Verdickung (18) aufweisen, an welche sich eine kegelförmige Spitze (19,20) anschließt.

6. Elektrodenarray nach einem der Ansprüche 1 bis 5, **dadurch gekennzeichnet, dass** die Nadelspitze (20) der Elektroden einen Durchmesser von 0,02-0,04mm aufweist.

7. Elektrodenarray nach einem der Ansprüche 1 bis 6, **dadurch gekennzeichnet, dass** die Elektroden (15) wenigstens eine erste und eine zweite Gruppe von Elektroden (15) aufweisen, wobei jede Gruppe von Elektroden mit einem eigenen elektrischen Anschluss elektrisch leitend verbunden ist.

8. Elektrodenarray nach einem der Ansprüche 1 bis 7, **dadurch gekennzeichnet, dass** das Elektrodenarray (5) aus einem elektrisch leitenden Material, wie z.B. Titan oder Platin-Iridium oder aus einem leitfähigen Kunststoff, wie z.B. Poly-3,4-ethylendioxythiophen, besteht.

9. Elektrodenarray nach einem der Ansprüche 1 bis 7, **dadurch gekennzeichnet, dass** die Elektroden (15) aus einem elektrisch leitenden Material, wie z.B. Titan oder Platin-Iridium oder aus einem leitfähigen Kunststoff, wie z.B. Poly-3,4-ethylendioxythiophen, bestehen und der Träger (14) aus einem elektrisch nicht-leitenden Material, insbesondere einem Polymer, besteht.

10. Elektrodenarray nach einem der Ansprüche 1 bis 7, **dadurch gekennzeichnet, dass** das Elektrodenarray (5) aus einem elektrisch nicht-leitenden Material besteht und die Elektroden (15) mit einer elektrisch leitenden Beschichtung versehen sind.

11. Elektrodenarray nach einem der Ansprüche 1 bis 10, **dadurch gekennzeichnet, dass** der Träger (14) als flexible Folie oder Blech ausgebildet ist.

12. Elektrode für die periphere Nervenstimulation, insbesondere für die aurikuläre Punktualstimulation z.B. des Vagusnerv, **dadurch gekennzeichnet, dass** die Elektrode (15) in einem mittleren Bereich eine Verdickung (18) aufweist, an welche sich eine kegelförmige Spitze (19,20) anschließt.

13. Elektrode nach Anspruch 12, **dadurch gekennzeichnet, dass** die Elektrode einen insbesondere zylindrischen Basisabschnitt (17) aufweist, dessen Durchmesser geringer ist als der des verdickten mittleren Bereichs (18).

14. Vorrichtung zur peripheren Nervenstimulation, insbesondere zur aurikulären Punktualstimulation z.B. des Vagusnerv eines Patienten, umfassend einen Stromgenerator (1) zur Erzeugung von Stimulationsstromimpulsen und wenigstens ein Elektrodenarray (5,6,7) nach einem der Ansprüche 1 bis 11 oder wenigstens zwei Elektroden (15) nach Anspruch 12 oder 13, das bzw. die mit jeweils wenigstens einer elektrischen Leitung (2,3,4) mit dem Stromgenerator (1) verbunden ist bzw. sind.

15. Vorrichtung nach Anspruch 14, **dadurch gekennzeichnet, dass** der Stromgenerator (1) zur Erzeugung der Stimulationsstromimpulse mit einer Pulsfrequenz von < 1kHz und mit einer Stromamplitude von > 5mA ausgebildet ist.
